# EUROPÄISCHE PATENTSCHRIFT

(11) **EP 2 575 843 B1**
(45) Veröffentlichungstag und Bekanntmachung des Hinweises auf die Patenterteilung: **01.03.2017**
(21) Anmeldenummer: 11721514.5
(22) Anmeldetag: 20.05.2011
(51) Int. Cl.: A61K 31/352, A61K 31/704, A61K 31/7048, A61K 36/87

(54) **VERFAHREN ZUR HERSTELLUNG EINES ANGEREICHERTEN EXTRAKTES AUS BLÄTTERN VON VITIS VINIFERA L**
METHOD FOR PRODUCING AN ENRICHED EXTRACT FROM VITIS VINIFERA L. PLANTS
PROCÉDÉ DE FABRICATION D'UN EXTRAIT ENRICHI À PARTIR DE FEUILLES DE VITIS VINIFERA L

(30) Priorität: 28.05.2010 EP 10164316
(43) Veröffentlichungstag der Anmeldung: 10.04.2013
(73) Patentinhaber: Boehringer Ingelheim International GmbH, 55216 Ingelheim am Rhein (DE)
(72) Erfinder: SCHEURING, Uwe, 55216 Ingelheim Am Rhein (DE); LANGER, Martin, 55216 Ingelheim Am Rhein (DE); PLOHMANN, Bernd, 55216 Ingelheim Am Rhein (DE); FEISTEL, Bjoern, 56626 Andernach (DE); WALBROEL, Bernd, 56626 Andernach (DE)
(74) Vertreter: Catillon, Marie
(86) Internationale Anmeldenummer: PCT/EP2011/058282
(87) Internationale Veröffentlichungsnummer: WO 2011/147754

(56) Entgegenhaltungen:
- EP-A1- 1 762 234
- EP-A2- 0 348 781
- WO-A1-01/28363
- WO-A2-2004/047748
- FR-A1- 2 795 964
- US-A- 5 912 363

## Beschreibung

Die vorliegende Erfindung betrifft Extrakte aus Blättern von *Vitis vinifera L*., ihre Herstellung und Verwendung.

Unter der Bezeichnung Venenschwäche wird umgangssprachlich ein Krankheitsbild verstanden, bei dem es zu Wassereinlagerungen in den unteren Gliedmaßen kommt und sich durch Befindlichkeitsstörungen wie schweren und schnell ermüdenden Beinen äußert. Das sogenannte Versacken von Wasser in den Beinen bezeichnet eine ödematöse Ansammlung von Wasser im Gewebe und setzt voraus, dass Wasser aus den Blutgefäßen übertreten kann. Es ist demnach anzunehmen, dass die Permeabilität der Gefäße übermäßig gesteigert ist.

Die Venenschwäche kann als ein frühes Stadium der Chronischen Venösen Insuffizienz (CVI) gesehen werden. Die CVI, auch chronisch-venöses Stauungssyndrom oder Chronische Veneninsuffizienz genannt, beruht auf einer Mikrozirkulationsstörung der Gefäße infolge einer venösen Abflussbehinderung. Das Risiko, an einer chronisch-venösen Insuffizienz zu leiden, steigt mit Alter, Fettleibigkeit, einer Anamnese mit Venenentzündungen, tiefer Venenthrombose und schwerem Beintrauma. CVI kommt bei Frauen doppelt so häufig vor wie bei Männern.

Wichtig für die Entstehung einer chronisch-venösen Insuffizienz ist das Vorhandensein eines pathologischen Bluthochdrucks der oberflächlichen Venen. Normal ist dieser bei 20 - 30 mmHg, steigt jedoch durch venöse Thrombose, primär oder sekundär pathologische Venenklappen oder abgeschwächte Muskelpumpe auf 60-90 mmHg. Dieser Hochdruck verursacht die für die späteren Komplikationen verantwortlichen anatomischen, physiologischen und histologischen Veränderungen der Gefäße. Unter physiologischen Bedingungen wird das Kapillarbett vor starken Druckveränderungen geschützt, da präkapilläre Arteriolen sich reflektorisch kontrahieren können. Diesen Reflex scheinen Patienten mit chronisch erhöhtem venösen Druck jedoch verloren zu haben, d.h. dass bei Veränderungen der Körperlage Druckveränderungen direkt an das Kapillarbett weiter gegeben werden.

Bei dauerhaft erhöhtem venösen Druck entstehen Veränderungen, die die Kapillarwände betreffen. Dazu gehören eine Verbreiterung und Verlängerung des Kapillarbetts, erhöhte Endotheloberfläche, erhöhte Einlagerung von Kollagen IV in die Basalmembran und perikapilläre Einlagerung von Fibrin. Die Zusammenwirkung von erhöhtem venösen Druck und abnormen Kapillaren mit erhöhter Permeabilität führen zu einem Ausschwemmen von Wasser, großen Proteinen und roten Blutzellen in das Interstitium. Das sich ansammelnde Fibrin scheint auch weniger einfach gelöst werden zu können. Gleichzeitig kommt es zu einem verminderten Gasaustausch, also einer kutanen Hypoxie. Dies führt unter anderem zu einer Leukozytenaktivierung.

Einen ähnlichen Prozess kennt man aus der Akut-Phase-Reaktion von Entzündungen. Hierbei wird die Permeabilität der Gefäßwände durch Gefäßmediatoren, wie Histamin, Prostaglandine (z.B. PGE-2), Kinine und Serotonin, für wenige Minuten gesteigert. Typische Auslöser einer solchen Akut-Phasen-Reaktion sind Zytokine wie Interleukin-1 (IL-1ß), Interleukin-6 (IL-6), TumornekrosefaktorAlpha (TNF-α), Tumorwachstumsfaktor Beta (TGF-ß) oder Interferon-Gamma die unter anderem von Endothelzellen, Fibroblasten, Makrophagen, Granulozyten und Lymphozyten freigesetzt werden. Diese immunkompetenten Zellen setzen die Zytokine beispielsweise nach der Beschädigung oder dem Absterben benachbarter Zellen frei. Die Zytokine gelangen über die Blutbahn in die Leber wo sie zusammen mit Cortisol das Organ zur Produktion von etwa 30 verschiedenen Akut-Phasen-Proteinen (APP) anregt. Die APPs begünstigen die Wundheilungsprozesse und wirken der Gewebeschädigung entgegen. Die Auswirkungen der Akut-Phase-Reaktion sind sowohl lokal, als auch systemisch. Die lokalen Effekte sollen mögliche Infektionen begrenzen und Xenobiotika (zum Beispiel synthetisch hergestellte Farbstoffe, Pestizide und chlorierte Lösungsmittel) eliminieren. Tote oder geschädigte Zellen werden per Phagozytose beseitigt und Reparaturprozesse initiiert. Dagegen sind die systemischen Auswirkungen Abwehrreaktionen, die das Überleben des Gesamtorganismus sicherstellen sollen, beispielsweise gegen Endotoxine.

Entsprechend der Leitlinien der Deutschen Gesellschaft für Phlebologie (AWMF-Leitlinien-Register Nr. 037/009) werden unter anderem als adjuvante Pharmakotherapeutika in der Behandlung der Chronischen Venösen Insuffizienz folgende Mittel eingesetzt: Acetylsalicylsäure, Diuretika, Fibrinolytika (Defibrotide, Stanazolol, Sulodexide), Fibrinolyse-Verstärker, Iloprost, Pentoxyphyllin, Prostaglandin E1, Saponine (Extrakte aus Centella, Hippocastanus, Ruscus), Benzaron, Calcium-Dobesilat, Naftazone, Tribenoside, Ginkgo biloba sowie zahlreiche Substanzen, die der großen Gruppe der Flavonoide angehören [Havsteen BH. the biochemistry and medical significance of the flavonoids. Pharmacol Ther 2002;96(2-3):67-202]. Ein eindeutig positiver Effekt der Flavonoide auf die Ödementwicklung konnte nachgewiesen werden [Martinez MJ, Bonfill X, Moreno RM, Vargas E, Capellà D. Phlebotonics for venous insufficiency. Cochrane Database of Systematic Reviews 2005, Issue 3. Art. No.: CD003229. DOI: 10.1002/14651858.CD003229.pub2.].

Es hat sich gezeigt, dass in Weinbauregionen das Krankheitsbild der Venenschwäche deutlich geringer ausfällt. Dies legt nahe, dass in der Pflanze *Vitis vinifera* L. Verbindungen enthalten sein müssen, welche antagonistisch der Erkrankung entgegenwirken. Traditionell wurden Weinblätter sowohl als Gemüse gegessen, als auch in Form von Dekokten und Infusen medizinisch angewendet bei menopausalen Störungen oder zur Behandlung von Hämorriden. In der französischen Pharmakopoe ist ein Extrakt aus Rotweinlaub als Venentonikum empfohlen. Seit einigen Jahren sind unter dem Namen Antistax^{®} Präparate mit wässrigem Extrakt aus rotem Weinlaub im Markt, die erfolgreich bei Chronisch-venöser Insuffizienz (CVI) eingesetzt werden [Kiesewetter H, Koscielny J, Kalus U, Vix JM, Peil H, Petrini O, et al. Efficacy of orally administered extract of red vine leaf AS 195 (folia vitis viniferae) in chronic venous insufficiency (stages I-II). A randomized, double-blind, placebocontrolled trial. Arzneimittel-Forschung 2000;50(2):109-117]. Der hierin enthaltene Extrakt ist durch einen Gehalt von etwa 5% Flavonoiden gekennzeichnet. Als eine weitere Gruppe phenolischer Verbindungen sind etwa 0,1% Anthocyane enthalten.

Entsprechend der guten Verträglichkeit der bisher bekannten Weinlaubextrakte, war es Ziel der vorliegenden Erfindung einen neuartigen Extrakt bereitzustellen, der sich ebenso für eine Verwendung im gesamten Bereich entzündlicher Erkrankungen eignet. Lokale antiinflammatorische Effekte können gegebenenfalls entlang des gesamten gastrointestinalen Traktes hilfreich bei der Behandlung oder Prophylaxe entzündlicher Erkrankungen sein. Gingivitis, Parondontitis, Stomatitis, Ösophagitis und Gastritis beschreiben die entzündlichen Erkrankungen des oberen Teils des Verdauungstraktes. Lymphozytäre Colitis, Colitis ulcerosa und Morbus Crohn sind die bekanntesten Vertreter entzündlicher Erkrankungen des Intestinalbereiches.

Unter Hämorrhoiden versteht man umgangssprachlich die Krampfader-ähnliche oder auch knotenförmige Schwellung bzw. Erweiterung eines arteriovenösen Gefäßpolsters, das zwischen dem Enddarm und dem Schließmuskel des Afters liegt. Dieses Gefäßpolster wird auch als Plexus hämorrhoidalis bezeichnet. Es besteht aus einem Arterien- und Venengeflecht, das gemeinsam mit dem Schließmuskel den Feinverschluss des Afters ermöglicht. Wenn von Hämorrhoiden gesprochen wird, sind damit aber meist Hämorrhoidalleiden gemeint. Typische Symptome sind leichte Blutungen, Druckgefühl, Juckreiz, Hautausschlag und im fortgeschrittenen Stadium Probleme beim kontrollierten Abgang von Darmgasen und Stuhl. Ursachen können u.a. Krampfadern-ähnliche Aussackungen anusnaher Blutgefäße sein. Eine ähnliche Erkrankung mit der Bezeichnung Perianalthrombose (= unechte Hämorrhoiden) entsteht durch Ausbildung eines Blutgerinnsels (Thrombus) in den Venen der Analhaut und wird auch Analthrombose genannt. Perianalthrombosen entstehen oft durch langes Sitzen, dem Sitzen auf kalten Flächen, durch starkes oder langes Pressen beim Stuhlgang (z. B. bei hartem Stuhl) sowie während der Schwangerschaft und der Geburt. Sie können auch durch starken Husten, körperliche Belastung, Flüssigkeitsmangel durch zu wenig trinken und sogar durch Niesen hervorgerufen werden. Auch hierbei können Blutgefäße über das übliche Maß hinaus gedehnt werden. Sowohl bei Hämorrhoidalleiden, als auch bei Perianalthrombose ist neben operativen Maßnahmen die Gabe von Suppositorien mit schmerzstillenden oder antientzündlichen Wirkstoffen gängig (Leitlinie zu Hämorroidalleiden "www.uniduesseldorf.de/AWMF/II/081-007p.htm" und Leitlinie zu Analthrombose "www.derma.de/585.0.html").

### Stand der Technik

Für andere Pflanzenteile von *Vitis vinifera L*., beispielsweise den Traubenkernen sind bereits einige Anreicherungsmethoden beschrieben. Während Verfahren wie die Mikrowellenextraktion (CN 1102589C) aber nur für geringe Anreicherungsfaktoren durch Mehrausbeute sorgen, sind Extraktionen mit überkritischen Fluiden (CN 1107110C) nur für lipophile Strukturen geeignet. CN 1454896 verwendet eine saure Primärextraktion in Verbindung mit einer Flüssig-Flüssig-Extraktion zur Anreicherung oligomerer Proanthocyanidine. EP 348781 beschreibt ein Anreicherungsverfahren für oligomere phenolische Verbindungen aus Traubenkernen mittels einer Membranfiltration, während WO 2005036988 und EP 1909750 ein chromatographisches Verfahren zur selektiven Anreicherung von Polyphenolen und Abreicherung von Monomeren beanspruchen.

GB 934554 beschreibt eine warme alkoholisch-wässrige Extraktion (50-80% EtOH) von Rotweinlaub, sowie eine anschließende Ansäuerung zum Erhalt der roten Farbe. Zur Entfernung von Chlorophyll und Wachsen wird eine Flüssig-Flüssig-Extraktion mittels Benzol oder Tetrachlorkohlenstoff vorgeschlagen, alternativ eine Umfällung in reinem Wasser. Durch Aussalzen kann die Präzipitation der Gerbstoffe beschleunigt werden. Es folgt ein Aufreinigungsschritt über eine Flüssig-Flüssig-Extraktion mit Butanol oder Pentanol. Aus der resultierenden alkoholischen Extraktphase wird optional mit Ether, Petroleum oder Benzol eine Procyanidin-Fraktion ausgefällt und anschließend schonend getrocknet (im Vakuum oder auf einem Sprühturm). Dieses Verfahren ist sehr aufwendig und kostenintensiv und das resultierende Produkt hat sehr starke Abweichungen vom Referenzextrakt. Daher wird ein alternativer Herstellprozess gesucht, der eine kostengünstigere Herstellung erlaubt.

Aufgabe der vorliegenden Erfindung war es, alternative wirksame Extrakte aus Weinlaub bereitzustellen.

Gelöst wird die Aufgabe durch ein Verfahren zur Herstellung eines Extraktes aus Blättern von *Vitis vinifera L.* umfassend folgende Schritte:
a) Bereitstellen einer Droge aus Blättern von *Vitis vinifera L.*
b) Extraktion der Droge mit einem Eluenten ausgewählt aus Wasser und Gemischen von Wasser mit Alkohol, unter Erhalt eines Rohextraktes,
c) Abtrennen des Rohextraktes von der extrahierten Droge,
d) zumindest teilweises Entfernen des Eluenten, unter Erhalt Dickextraktes,
e) Rücklösen des Dickextraktes in Wasser,
f) Abtrennen unlöslicher Bestandteile,
g) selektive Anreicherung sekundärer Pflanzeninhaltsstoffe durch
   i. eine 2-Phasenextraktion oder
   ii. eine Membranfiltration.

Erfindungsgemäß werden Blätter von *Vitis vinifera L.* eingesetzt. Diese können prinzipiell frisch oder getrocknet eingesetzt werden.

Es wird bevorzugt, dass die Blätter vor der Extraktion zerkleinert werden.

In einer speziellen Ausführungsform der vorliegenden Erfindung erfolgt die Bereitstellen der Droge durch Sammeln von Blättern von *Vitis vinifera L.* zu einem Zeitpunkt, an dem der Flavonoidgehalt ein Optimum erreicht, Trocknen und Zerkleinern der Blätter sowie optional durch Schneiden der Blätter in Stücke.

Die Extraktion erfolgt mit Wasser oder Gemischen von Wasser mit Alkohol, um einen Rohextrakt zu erhalten. Besonders geeignet sind Mengen von 5 bis 30 kg Extraktionsmittel pro 1 kg getrockneter Droge.

Bevorzugt wird als Eluent Wasser oder eine Ethanol-Wasser-Mischung eingesetzt, die 5 bis 95% (V/V) Ethanol enthält.

Besonders bevorzugt ist der Einsatz eines Eluenten, der neben Wasser, Ethanol in einer Menge von 25 bis 50% (V/V) enthält.

Bevorzugt wird die Extraktion bei einer Temperatur im Bereich von 40 bis 80° durchgeführt, wobei sich ein Temperaturbereich von 15 bis 25°C unterhalb der Siedetemperatur des verwendeten Eluenten besonders bewährt hat.

Der bei der Extraktion vorherrschende Druck ist über weite Bereiche unkritisch. Üblicherweise wird man die Extraktion jedoch bei einem Druck im Bereich von 900 bis 1500 mbar, bevorzugt von 950 bis 1100 mbar und insbesondere bei Umgebungsdruck, d.h. beietwa 1013 mbar, durchführen.

Der Rohextrakt wird von der extrahierten Droge abgetrennt. Das Abtrennen erfolgt üblicherweise durch Filtration oder andere dem Fachmann bekannte Verfahren. Beispielsweise eignet sich eine Filtration über eine Sieblochplatte mit 4mm-Löchern und nachgeschalteten Polyamid-Siebbeuteln mit 250µm Maschenweite. Weitere geeignete Verfahren hierfür sind dem Fachmann bekannt.

Anschließend wird der Eluent aus dem abgetrennten Rohextrakt zumindest teilweise unter Erhalt eines Dickextraktes entfernt. Unter teilweiser Entfernung des Eluenten ist im Rahmen der vorliegenden Erfindung eine Entfernung des Eluenten von 30 bis 99 % (V/V) bezogen auf das eingesetzte Eluentenvolumen, bevorzugt 75 bis 98 % (V/V) und insbesondere eine im Wesentlichen vollständige Entfernung des Eluenten zu verstehen, d.h. eine Entfernung von wenigstens 95 % (V/V). Üblicherweise wird man den Eluenten durch Erwärmen und/oder unter vermindertem Druck entfernen. Geeignete Methoden und Vorrichtungen hierfür sind dem Fachmann bekannt.

Der so erhaltene Dickextrakt wird in Wasser rückgelöst und unlösliche Bestandteile werden abgetrennt.

Üblicherweise erfolgt die Rücklösung in einer Menge von 1 Teil bis 10 Teilen Wasser bezogen auf eine Menge von 1 Teil des erhaltenen Rohextrakts. Die unlöslichen Bestandteile werden durch fachübliche Methoden, wie Filtration und/oder Zentrifugieren abgetrennt.

Anschließend erfolgt eine selektive Anreicherung sekundärer Pflanzeninhaltsstoffe entweder durch eine 2-Phasen-Extraktion oder eine Membranfiltration.

Die 2-Phasen-Extraktion kann als Fest-Phasen-Extraktion durchgeführt werden, wobei sich als feste Phase ein Adsorberharz bewährt hat. Geeignete Adsorberharze sind beispielsweise aus der Gruppe der nichtionischen hydrophoben Divinylbenzen-Copolymere, aliphatischen Esterpolymere und Formophenolpolymere unter den Bezeichnungen Amberlite^{®}, Levatite^{®}, Miontech^{®} und Diaion^{®} kommerziell verfügbar. Die für das erfindungsgemäße Verfahren geeigneten Adsorberharze sind bevorzugt durch eine durchschnittliche innere Porengröße im Bereich von 300 - 700 Angström, bevorzugt von 400 - 500 Angström, gekennzeichnet.

Die Substanzen, die an den Adsorber binden, werden eluiert und weiter verwendet; die nicht gebundenen Substanzen werden verworfen.

Üblicherweise wird man die Extraktion in einem Temperaturbereich von 10 bis 30 °C durchführen. Die verwendete Aufgabelösung wird üblicherweise zwischen pH 3 und pH 6 eingestellt, bevorzugt zwischen pH 4,5 und pH 5,5, besonders bevorzug auf pH 5.

Es kann sich bei der 2-Phasen-Extraktion auch um eine Flüssig-Flüssig-Extraktion handeln, wobei bevorzugt die zweite Phase eine mit Wasser nicht mischbare Flüssigkeit ist und ausgewählt aus der Gruppe der Alkohole, Ketone, Alkane oder Ester. In einer speziellen Ausführungsform ist die zweite Phase ein mit Wasser nicht mischbarer Alkohol, bevorzugt ein C₄-C₅-Alkohol und besonders bevorzugt n-Butanol. Hierbei wird die Fraktion, die in die zweite Phase übertritt weiterverwendet und die wässrige Phase verworfen.

Üblicherweise wird man die Extraktion mit einem Trockensubstanzgehalt der Ausgangslösung zwischen 3 und 30% durchführen, bevorzugt zwischen 10 und 20%, besonders bevorzugt bei 15%.

Grundsätzlich kann es sich bei der selektiven Anreicherung auch um eine Membranfiltration handeln, wobei sich Membranen mit einer Ausschlussgröße von 30 bis 2000 Kilodalton(kDa) bewährt haben.

Das Permeat wird weiter verwendet, während das Retentat verworfen wird.

In einer speziellen Ausführungsform der vorliegenden Erfindung wird man den Extrakt nach erfolgter selektiver Anreicherung unter Erhalt eines Trockenextraktes von *Vitis vinifera L.* trocknen. Die Trocknung erfolgt unter Verwendung von fachüblichen Verfahren, wie z.B. der Sprühbandtrocknung. Es ist jedoch ebenso möglich den angereicherten Extrakt ohne vorherige Trocknung weiterzuverwenden.

Durch das zuvor beschriebene erfindugsgemäße Verfahren werden Extrakte von Vitis vinifera L. erhalten, deren Verhältnis von nativer Droge zu Extrakt (DEVnativ) 12 : 1 bis 40 : 1 beträgt. Der Gesamtflavonoidgehalt der erfindungsgemäß erhaltenen und erfindungsgemäßen Extrakte beträgt üblicherweise wenigstens 15 Gew.-%, bevorzugt wenigstens 20 Gew.-%, und der Gehalt an Anthocyanen wenigstens 0,1 Gew.-%, bevorzugt wenigstens 0,2 Gew.-%, besonders bevorzugt wenigstens 0,3 Gew.-%. Hierdurch wird eine verstärkte Inhibierung Permeabilitäts-triggernder Zytokine im Vergleich zum Referenzextrakt (gemäß Stand der Technik) erreicht.

Demzufolge ist ein weiterer Gegenstand der vorliegenden Erfindung der Extrakt hergestellt nach einem der zuvor beschriebenen Verfahren.

Ein weiterer Gegenstand der vorliegenden Efindung betrifft eine Extraktzubereitung, enthaltend einen erfindungsgemäßen Extrakt sowie mindestens einen physiologisch verträglichen Hilfsstoff. Geeignete physiologisch verträgliche Hilfsstoffe sind dem Fachmann bekannt.

Ein weiterer Gegenstand der vorliegenden Efindung betrifft die Verwendung eines erfindungsgemäßen Extrakts oder einer erfindungsgemäßen Extraktzubereitung, zur Herstellung von Medikamenten, pharmazeutischen Zubereitungen oder Nahrungsergänzungsmitteln. Die Herstellung erfolgt nach fachüblichen Verfahren.

Ein weiterer Gegenstand der vorliegenden Efindung betrifft die Verwendung eines erfindungsgemäßen Extrakts oder einer erfindungsgemäßen Extraktzubereitung zur Behandlung oder Prophylaxe von entzündlichen Erkrankungen der Blutgefäße oder des Gastrointestinaltraktes, bei venöser Mikrozirkulationsstörung, bei ischämisch bedingten Erkrankungen, bei Hämorrhoidalleiden oder bei Perianalthrombose. Bei den erfindungsgemäß behandelten entzündlichen Erkrankung der Blutgefäße handelt es sich insbesondere um Phlebitis. Bei den erfindungsgemäß behandelten venösen Mikrozirkulationsstörung handelt es sich insbesondere um eine Chronisch venöse Insuffizienz (CVI). Bei den erfindungsgemäß behandelten ischämisch bedingten Erkrankungen handelt es sich insbesondere um Erkrankungen des Herzens, speziell um Herzinfarkt.

Ein weiterer Gegenstand der vorliegenden Efindung betrifft die Verwendung eines erfindungsgemäßen Extrakts oder einer erfindungsgemäßen Extraktzubereitung zur Behandlung oder Prophylaxe von akuten oder chronischen Entzündungen des gastrointestinalen Traktes oder Hämorrhoidalleiden oder Perianalthrombose.

Die erfindungsgemäßen Verwendungen zur Behandlung nach können intravasale, perorale, rektale oder transdermael Verabreichung des erfindungsgemäßen Extrakts oder der erfindungsgemäßen Extraktzubereitung erfolgen. Dabei weisen die erfindungsgemäßen Extrakte oder erfindungsgemäßen Extraktzubereitungen topische oder systemische Wirkung auf.

Die Erfindung wird durch die nachfolgenden nicht einschränkenden Beispiele näher erläutert.

### Verwendete Messmethoden

### Chemische Parameter

Die Gehaltsbestimmung der Flavonoide erfolgt mittels einer HPLC-Methode, welche die Flavonoide Isoquercitrin, Quercetin-3-O-ß-D-glucuronid und Kämpferol-3-O-ß-D-glucosid erfasst. Als stationäre Phase dient hierbei eine Superspher RP8 Trennsäule, mit 4 µm Partikeln, in der Dimension 125 x 4 mm. Die isokratische Trennung erfolgt bei einer Flussgeschwindigkeit von 1 ml/min in einem Säulenofen bei 25 °C. Der Eluent setzt sich aus 107 Teilen Tetrahydrofuran, 55 Teilen Acetonitril und 810 Teilen Phosphorsäure (0,003 mol/l) zusammen. Die Detektion erfolgt bei 360 nm.
Der Gehalt an Anthocyanen wird mit einer separaten HPLC-Methode analog Ph.Eur 6.4 Monographie 2394 "Fresh Bilberry fruit dry extract, refined and standardised" ermittelt.

### Pharmakologisches in-vitro-Modell zur Zytokin-Inhibierung

Humane Monozyten werden bei 37 °C und 5 % CO₂ für 24 h mit LPS (10ng/ml) stimuliert. Die Extrakte werden 30 Minuten vor Zugabe des LPS zugegeben, um zu prüfen, ob diese die LPS-Stimulation beeinflussen können. Es wurden Messkonzentrationen der Extrakte zwischen 10 - 300 µg/ml getestet. Nach 24 Stunden wurden die überstehenden Lösungen abgenommen, zentrifugiert und auf die Konzentrationen an PGE-2, IL-6, IL-1, und TNF-alpha mittels ELISAs / EIAs (Biotrend / Immunotools) gemäß Herstellervorgaben vermessen.

### Beispiel 1: wässriger Referenzextrakt (Stand der Technik)

2 kg Droge Folia Vitis vinifera Ph.Eur. werden mit 24 Litern Osmosewasser bei 75°C im Perkolator extrahiert. Das Eluat wird filtriert und im Unterdruck zu einem Dickextrakt mit ca. 50% Trockensubstanzanteil eingeengt. Der Spissum wird mit 4% Siliciumdioxid versetzt und bei 50°C im Vakuumtrockenschrank getrocknet. Der erhaltene Extrakt ist charakterisiert durch ein DEVnativ von 5 : 1, einen Flavonoidgehalt von 5,1 % und einen Gehalt an Anthocyanen von 0,15 %.

| [%] Inhibierung der Ausschüttung von | | | |
|---|---|---|---|
| TNF-alpha | IL-1ß | IL-6 | PGE-2 |
| 50,4 ± 5,9 bei 50 µg/ml | 46,9 ± 1,9 bei 300 µg/ml | 42,5 ± 5,3 bei 50 µg/ml | 11,6 ± 5,5 bei 300 µg/ml |

### Beispiel 2: erfindungsgemäßer Adsorberextrakt (Basis wässrig)

2 kg Droge Folia Vitis vinifera Ph.Eur. werden mit 44 Litern Osmosewasser bei 75°C im Perkolator extrahiert. Das Eluat wird im Unterdruck zu einem Dickextrakt mit ca. 50% Trockensubstanz-Anteil eingeengt. Der Spissum wird über 16 Stunden kühl gelagert, und mit Osmosewasser auf einen Trockensubstanzanteil von 10% zurück verdünnt. Nach 24 Stunden werden die ausgefallenen Gerbstoffe (Tannine) abfiltriert und anschließend der Dünnextrakt auf Adsorbermaterial XAD7HP aufgebracht. Der beladene Adsorber wird mit Osmosewasser gewaschen und anschließend die Extraktkomponente mit Ethanol eluiert. Die Ethanolphase wird auf einen Trockensubstanzanteil von >50% eingedampft. Der erhaltene Spissum wird nativ bei 50°C im Vakuumtrockenschrank getrocknet. Der erhaltene Extrakt ist charakterisiert durch ein DEVnativ von 19 : 1, einen Flavonoidgehalt von 21,1 % und einen Gehalt an Anthocyanen von 0,77 %.

| [%] Inhibierung der Ausschüttung von | | |
|---|---|---|
| Zytokin | IL-6 | PGE-2 |
| Extrakt gemäß Bsp. 2 | 42,5 ± 4,6 bei 10 µg/ml | 32,1 ± 7,1 bei 50 µg/ml |
| Extrakt gemäß Bsp. 1 | 42,5 ± 5,3 bei 50 µg/ml | 11,6 ± 5,5 bei 300 µg/ml |

Für den Adsorberextrakt nach Beispiel 2 konnte eine Steigerung der Zytokin-Inhibierung gegenüber dem Stand der Technik nach Bsp. 1 nachgewiesen werden.

Bei IL-6 ist eine um den Faktor 5 geringere Extraktkonzentration des erfindungsgemäßen Extraktes notwendig, um den gleichen anti-inflammatorischen Effekt zu erzielen.

Die Beeinflussung von PGE-2 ist noch stärker ausgeprägt. Bei einer Mess-konzentration von 300µg/ml nach Bsp. 1 konnte nur eine 11,6%ige Inhibierung gemessen werden. Der erfindungsgemäße Extrakt nach Bsp. 2 erreichte mit einer 6-fach niedrigeren Konzentration von 50 µg/ml sogar eine 32,1 %ige Inhibierung.

Diese beiden um den Faktor 5 bzw. Faktor 18 (3fach besserer %-Wert x Konzentrationsfaktor 6) liegen über dem Anreicherungsfaktor von 3,8 des erfindungsgemäßen Extraktes vs. Bsp. 1. Der Anreicherungsfaktor von 3,8 ergibt sich aus dem Quotienten des DEV nativ der zu vergleichenden Extrakte.

### Beispiel 3: erfindungsgemäßer Extrakt (mittels FI.-FI. Extraktion)

2 kg Droge Folia Vitis vinifera Ph.Eur. werden mit 44 Litern Osmosewasser bei 75°C im Perkolator extrahiert. Das Eluat wird im Unterdruck zu einem Dickextrakt mit ca. 50% Trockensubstanz-Anteil eingeengt. Der Spissum wird über 16 Stunden kühl gelagert, und mit Osmosewasser auf einen Trockensubstanzanteil von 10% zurück verdünnt. Nach 24 Stunden wird die ausgefallene Tanninfraktion abfiltriert und anschließend der Dünnextrakt zweimal mit n-Butanol im Verhältnis 3:2 unterzogen. Die Butanolphase wird auf einen Trockensubstanzanteil von >50% eingedampft. Der erhaltene Spissum wird nativ bei 50°C im Vakuumtrockenschrank getrocknet. Der erhaltene Extrakt ist charakterisiert durch eine DEVnativ von 16:1, einen Flavonoidgehalt von 21,7 % und einen Gehalt an Anthocyanen von 0,43 %. Die verbliebene Wasserphase charakterisiert sich über ein DEVnativ von 5:1 einen Flavonoidgehalt von <0,1 % und einen Gehalt an Anthocyanen unterhalb der Bestimmungsgrenze.

| | [%] Inhibierung der Ausschüttung von | | |
|---|---|---|---|
| | TNF-alpha | IL-1ß | PGE 2 |
| BuOH-Phase Bsp. 3A | 52,2 ± 6,7 bei 10 µg/ml | 55,8 ± 6,6 bei 10 µg/ml | 24,3 ± 3,9 bei 50 µg/ml |
| H₂O-Phase Bsp.3B | 17,1 ± 4,8 bei 50 µg/ml | 39,8 ± 6,0 bei 300 µg/ml | 1,6 ± 4,6 bei 300 µg/ml |

Der Vergleich beider Phasen aus der Flüssig-Flüssig-Bearbeitung zeigt deutlich, dass die anti-inflammorischen Prinzipien in der organischen Phase (Butanol) angereichert werden können.

Für den butanolisch angereicherten Extrakt nach Beispiel 3A konnte eine Steigerung der Zytokin-Inhibierung von Faktor 15 (TNF-a), Faktor 42 (IL-1ß) und Faktor 91 (PGE-2) gegenüber der abgetrennten Wasserphase nach Bsp. 3B ermittelt werden Demgegenüber steht nur ein Anreicherungsfaktor von 3,2 (Quotient des DEV nativ der zu vergleichenden Extrakte Bsp. 3A vs. Bsp. 3B).

Dies unterstreicht die selektive Anreicherung der aktiven Prinzipien aus der Wasserphase in die organische Phase.

Auch ist der erfindungsgemäße Extrakt nach Bsp. 3A dem Stand der Technik überlegen.

| [%] Inhibierung der Ausschüttung von | | |
|---|---|---|
| Zytokin | TNF-alpha | PGE 2 |
| Extrakt gemäß Bsp. 3A | 52,2 ± 6,7 bei 10 µg/ml | 24,3 ± 3,9 bei 50 µg/ml |
| Extrakt gemäß Bsp. 1 | 50,4 ± 5,9 bei 50 µg/ml | 11,6 ± 5,5 bei 300 µg/ml |

Für den Extrakt nach Beispiel 3A konnte eine Steigerung der Zytokin-Inhibierung gegenüber dem Stand der Technik nach Bsp. 1 nachgewiesen werden.

Bei TNF-alpha ist eine um den Faktor 5 geringere Extraktkonzentration des erfindungsgemäßen Extraktes 3A notwendig, um den gleichen anti-inflam-matorischen Effekt zu erzielen.

Die Beeinflussung von PGE-2 ist noch stärker ausgeprägt. Bei einer Mess-konzentration von 300µg/ml nach Bsp. 1 konnte nur eine 11,6%ige Inhibierung gemessen werden. Der erfindungsgemäße Extrakt nach Bsp. 2 erreichte mit einer 6-fach niedrigeren Konzentration von 50 µg/ml sogar eine 24,3%ige Inhibierung.

Diese beiden um den Faktor 5 bzw. Faktor 12 (2fach besserer %-Wert x Konzentrationsfaktor 6) liegen über dem Anreicherungsfaktor von 3,2 des erfindungsgemäßen Extraktes vs. Bsp. 1. Der Anreicherungsfaktor von 3,2 ergibt sich aus dem Quotienten des DEV nativ der zu vergleichenden Extrakte.

### Beispiel 4: Herstellung eines erfindungsgemäßen ethanolischen Extraktes

2 kg Droge Folia Vitis vinifera Ph.Eur. werden mit 24 Litern EtOH 40% V/V bei 45°C im Perkolator extrahiert. Das Eluat wird filtriert und im Unterdruck zu einem Dickextrakt mit ca. 50% Trockensubstanzanteil eingeengt. Der Spissum wird über 16 Stunden kühl gelagert, und mit Osmosewasser auf einen Trockensubstanzanteil von 10% zurück verdünnt. Nach 24 Stunden werden die ausgefallenen Gerbstoffe abfiltriert und anschließend der Dünnextrakt mittels einer Membranfiltration (MWCO 300 kDa) von sonstigen koextrahierten hochmolekularen Substanzen befreit. Die resultierende Fraktion (Permeat) wird bei 50°C im Vakuum bis zur Trockne gebracht. Der erhaltene Extrakt ist charakterisiert durch ein DEVnativ von 6,7 : 1 und einen Flavonoidgehalt von 7,6 %.

### Beispiel 5: Herstellung eines erfindungsgemäßen Adsorberextraktes (Basis EtOH)

2 kg Droge Folia Vitis vinifera Ph.Eur. werden mit 24 Litern EtOH 20% V/V bei 75°C im Perkolator extrahiert. Das Eluat wird im Unterdruck zu einem Dickextrakt mit ca. 50% Trockensubstanzanteil eingeengt. Der Spissum wird über 16 Stunden kühl gelagert, und mit Osmosewasser auf einen Trockensubstanzanteil von 10% zurück verdünnt. Nach 24 Stunden werden die ausgefallenen Gerbstoffe abfiltriert und anschließend der Dünnextrakt auf Adsorbermaterial XAD7HP aufgebracht. Der beladene Adsorber wird mit Osmosewasser gewaschen und anschließend die Extraktkomponente mit Ethanol eluiert. Die Ethanolphase wird auf einen Trockensubstanzanteil von >50% eingedampft. Der erhaltene Spissum wird nativ bei 50°C im Vakuumtrockenschrank getrocknet. Der erhaltene Extrakt ist charakterisiert durch ein DEVnativ von 21 : 1 und einen Flavonoidgehalt von 25,1 %.

## Patentansprüche

1. Verfahren zur Herstellung eines Extraktes aus Blättern von *Vitis vinifera L.* umfassend folgende Schritte:
a) Bereitstellen einer Droge aus Blättern von *Vitis vinifera L.*
b) Extraktion der Droge mit einem Eluenten ausgewählt aus Wasser und Gemischen von Wasser mit Alkohol, unter Erhalt eines Rohextraktes,
c) Abtrennen des Rohextraktes von der extrahierten Droge,
d) zumindest teilweises Entfernen des Eluenten, unter Erhalt eines Dickextraktes,
e) Rücklösen des Dickextraktes in Wasser,
f) Abtrennen unlöslicher Bestandteile,
g) selektive Anreicherung sekundärer Pflanzeninhaftsstoffe durch
i. eine 2-Phasenextraktion oder
ii. eine Membranfiltration.

2. Verfahren nach Anspruch 1, wobei der Eluent Wasser oder eine Ethanol-Wassermischung ist, die 5 - 95 % (V/V) Ethanol enthält.

3. Verfahren nach einem der vorhergehenden Ansprüche, wobei der Eluent zur Extraktion auf eine Temperatur von 40 - 80°C erwärmt wird, bevorzugt auf 15 - 25°C unterhalb der Siedetemperatur.

4. Verfahren nach einem der vorhergehenden Ansprüche, wobei die 2-Phasenextraktion eine Fest-Phasen-Extraktion ist.

5. Verfahren nach Anspruch 4, wobei die zweite Phase ein Adsorberharz ist, bevorzugt ausgewählt aus der Gruppe der nichtionischen hydrophoben Divinylbenzen-Copolymere, aliphatischen Esterpolymere und Formophenolpolymere.

6. Verfahren nach einem der vorhergehenden Ansprüche, wobei die 2-Phasen-Extraktion eine Flüssig-Flüssig-Extraktion ist.

7. Verfahren nach Anspruch 6, wobei die zweite Phase eine mit Wasser nicht mischbare Flüssigkeit ist, ausgewählt aus der Gruppe der Alkohole, Ketone, Alkane oder Ester.

8. Extrakt, erhältlich nach einem Verfahren gemäss einem der Ansprüche 1 bis 7.

9. Extrakt nach Anspruch 8, wobei das Verhältnis von nativer Droge zu Extrakt (DEVnativ) 12 : 1 bis 40 : 1 beträgt, der Gesamtflavonoidgehalt wenigstens 15 Gew.-% beträgt und der Gehalt an Anthocyanen wenigstens 0,1 Gew.-% beträgt.

10. Extraktzubereitung, enthaltend einen Extrakt nach einem der Ansprüche 8 bis 9 sowie mindestens einen physiologisch verträglichen Hilfsstoff.

11. Verwendung eines Extrakts oder einer Extraktzubereitung nach einem der Ansprüche 8 bis 10, zur Herstellung von Medikamenten, pharmazeutischen Zubereitungen oder Nahrungsergänzungsmitteln.

12. Extrakt oder Extraktzubereitung nach einem der Ansprüche 8 bis 10 für die Verwendung zur Behandlung oder Prophylaxe von entzündlichen Erkrankungen der Blutgefäße oder des Gastrointestinaltraktes, bei venöser Mikrozirkulationsstörung, bei ischämisch bedingten Erkrankungen, bei Hämorrhoidalleiden oder bei Perianalthrombose.

13. Extrakt oder Extraktzubereitung nach Anspruch 12, wobei die entzündliche Erkrankung der Blutgefäße eine Phlebitis ist.

14. Extrakt oder Extraktzubereitung nach Anspruch 12, wobei die venöse Mikrozirkulationsstörung eine Chronisch venöse Insuffizienz (CVI) ist.

15. Extrakt oder Extraktzubereitung nach Anspruch 12, wobei die ischämisch bedingten Erkrankungen ausgewählt sind unter Erkrankungen des Herzens, insbesondere Herzinfarkt.

## Claims

1. Process for preparing an extract of leaves of *Vitis vinifera L.* comprising the following steps:
a) preparing a drug from leaves of *Vitis vinifera L.*
b) extracting the drug with an eluant selected from among water and mixtures of water with alcohol, to obtain a raw extract,
c) separating the raw extract from the extracted drug,
d) at least partly eliminating the eluant, to obtain a thick extract,
e) redissolving the thick extract in water,
f) separating off any insoluble constituents,
g) selectively concentrating secondary plant ingredients by
i. a 2-phase extraction or
ii. membrane filtration.

2. Process according to claim 1, wherein the eluant is water or an ethanol-water mixture that contains 5 - 95 % (V/V) ethanol.

3. Process according to one of the preceding claims, wherein for the extraction the eluant is heated to a temperature of 40 - 80°C, preferably to 15 - 25°C below the boiling temperature.

4. Process according to one of the preceding claims, wherein the 2-phase extraction is a solid phase extraction.

5. Process according to claim 4, wherein the second phase is an adsorber resin, preferably selected from among the group of non-ionic hydrophobic divinylbenzene copolymers, aliphatic ester polymers and formophenol polymers.

6. Process according to one of the preceding claims, wherein the two-phase extraction is a liquid-liquid extraction.

7. Process according to claim 6, wherein the second phase is a water-immiscible liquid selected from among the group of alcohols, ketones, alkanes or esters.

8. Extract obtainable according to a process according to one of claims 1 to 7.

9. Extract according to claim 8, wherein the ratio of native drug to extract (DEVnative) is 12:1 to 40:1, the total flavonoid content is at least 15 % by weight and the content of anthocyanins is at least 0.1 % by weight.

10. Extract preparation, containing an extract according to one of claims 8 to 9 and at least one physiologically acceptable adjuvant.

11. Use of an extract or of an extract preparation according to one of claims 8 to 10 for preparing medicaments, pharmaceutical preparations or nutritional supplements.

12. Extract or extract preparation according to one of claims 8 to 10, for use in the treatment or prophylaxis of inflammatory diseases of the blood vessels or of the gastrointestinal tract, in venous microcirculatory disorders, in ischaemic diseases, in haemorrhoidal complaints or in perianal thrombosis.

13. Extract or extract preparation according to claim 12, wherein the inflammatory disease of the blood vessels is phlebitis.

14. Extract or extract preparation according to claim 12, wherein the venous microcirculatory disorder is chronic venous insufficiency (CVI).

15. Extract or extract preparation according to claim 12, wherein the ischaemic diseases are selected from among the heart diseases, particularly myocardial infarct.

## Revendications

1. Procédé de fabrication d'un extrait de feuilles de *Vitis vinifera L.* comprenant les étapes suivantes :
a) la mise à disposition d'une drogue provenant des feuilles de *Vitis vinifera L.*
b) l'extraction de la drogue avec un éluant choisi parmi l'eau et des mélanges d'eau et d'alcool, en obtenant un extrait brut,
c) la séparation de l'extrait brut de la drogue extraite,
d) l'élimination au moins partielle de l'éluant, en obtenant un extrait épaissi,
e) la redissolution de l'extrait épaissi dans de l'eau,
f) la séparation des composants insolubles,
g) l'enrichissement sélectif des ingrédients végétaux secondaires par
i. une extraction biphasique ou
ii. une filtration membranaire.

2. Procédé selon la revendication 1, dans lequel l'éluant est l'eau ou un mélange éthanol - eau qui contient 5 à 95 % (v/v) d'éthanol.

3. Procédé selon l'une des revendications précédentes, dans lequel l'éluant destiné à l'extraction est chauffé à une température de 40 à 80 °C, de préférence de 15 à 25 °C en dessous de la température d'ébullition.

4. Procédé selon l'une des revendications précédentes, dans lequel l'extraction biphasique est une extraction en phases solides.

5. Procédé selon la revendication 4, dans lequel la deuxième phase est une résine adsorbante, de préférence choisie dans le groupe des copolymères de divinylbenzène hydrophobes non ioniques, des polymères d'esters aliphatiques et des polymères de formophénol.

6. Procédé selon l'une des revendications précédentes, dans lequel l'extraction biphasique est une extraction liquide - liquide.

7. Procédé selon la revendication 6, dans lequel la deuxième phase est un liquide non miscible avec l'eau, choisi dans le groupe des alcools, des cétones, des alcanes ou des esters.

8. Extrait susceptible d'être obtenu selon un procédé d'après l'une des revendications 1 à 7.

9. Extrait selon la revendication 8, dans lequel le rapport entre la drogue native et l'extrait (DEVnativ) est de 12:1 à 40:1, la teneur totale en flavonoïde est d'au moins 15 % en poids et la teneur en anthocyanène est d'au moins 0,1 % en poids.

10. Préparation d'extrait, contenant un extrait selon l'une des revendications 8 à 9 ainsi qu'au moins un excipient physiologiquement acceptable.

11. Utilisation d'un extrait ou d'une préparation d'extrait selon l'une des revendications 8 à 10, pour la fabrication de médicaments, de compositions pharmaceutiques ou de compléments alimentaires.

12. Extrait ou préparation d'extrait selon l'une des revendications 8 à 10 pour utilisation pour le traitement ou la prophylaxie des maladies inflammatoires des vaisseaux sanguins ou du tractus gastro-intestinal, en cas de troubles de la microcirculation veineuse, de maladies d'origine ischémique, de douleurs hémorroïdaires ou de thrombose périanale.

13. Extrait ou préparation d'extrait selon la revendication 12, dans lequel la maladie inflammatoire des vaisseaux sanguins est une phlébite.

14. Extrait ou préparation d'extrait selon la revendication 12, dans lequel le trouble de la microcirculation veineuse est une insuffisance veineuse chronique (IVC).

15. Extrait ou préparation d'extrait selon la revendication 12, dans lequel les maladies d'origine ischémique sont choisies parmi les maladies du coeur, en particulier l'infarctus du myocarde.
